# EUROPEAN PATENT APPLICATION

(11) **EP 4 545 107 A1**
(43) Date of publication of application: **30.04.2025**
(21) Application number: 22948950.5
(22) Date of filing: 26.09.2022
(51) Int. Cl.: A61L 27/40, A61L 24/04, A61B 18/20

(54) **PEG TWO-COMPONENT SELF-ADHESIVE ABSORBABLE BIOLOGICAL MESH, METHOD FOR PREPARING SAME, AND USE THEREOF**

(30) Priority: 27.06.2022 CN 202210744873
(71) Applicant: Beijing Biosis Healing Biological Technology Co., Ltd., Beijing 102600 (CN)
(72) Inventor: ZHAO, Bo, Beijing 102600 (CN); WEI, Pengfei, Beijing 102600 (CN); HUANG, Yiqian, Beijing 102600 (CN); JING, Wei, Beijing 102600 (CN); YU, Xueqiao, Beijing 102600 (CN); ZHANG, Yan, Beijing 102600 (CN)
(74) Representative: Perani & Partners S.p.A.
(86) International application number: PCT/CN2022/121487
(87) International publication number: WO 2024/000861

(57) **Abstract**

In view of the problems of inadequate adhesiveness, low operability, and inadequate convenience and portability of current tissue sealing materials, the present disclosure provides a PEG bicomponent self-adhesive absorbable biological patch, comprising a substrate layer and an adhesive coating located on the substrate layer, wherein the substrate layer is a biological material, and the adhesive coating is a hydrogel formed by a bicomponent of polyethylene glycol succinimidyl ester and polyethylene glycol amine at a specific ratio. The biological patch can be adhered firmly to the tissue surface and applied directly without extra preparation, and has good biocompatibility, has biodegradability, and can be used in common clinical scenarios such as tissue adherence, wound hemostasis, and low-pressure leakage of body fluids.

## Description

The present application claims the benefit of a priority of an earlier application filed with the China National Intellectual Property Administration on June 27, 2022, of which the Chinese Patent Application No. is 202210744873.8 entitled "PEG BICOMPONENT SELF-ADHESIVE ABSORBABLE BIOLOGICAL PATCH AND PREPARATION METHOD AND USE THEREOF". The entire contents of this earlier application are incorporated herein by reference.

### TECHNICAL FIELD

The present disclosure relates to the technical field of medical biomaterials, in particular to a polyethylene glycol-based bicomponent self-adhesive absorbable biological patch, and a preparation method and medical use thereof.

### BACKGROUND

Tissue sealing material is a material used to seal and block damaged areas in time when damage, bleeding, or low-pressure fluid leakage occurs to body tissues.

At present, the predominant tissue sealing materials in the market include a liquid state (such as Braun's Histoacryl products with the main component of butyl-2-cyanoacrylate), a semi-solid state (such as Ethicon's Surgiflo products with the main component of porcine-derived gelatin), and a powder state (such as Baxter's Coseal products with the main component of two modified polyethylene glycol polymers).

Histoacryl is an n-butyl α-cyanoacrylate product. Its mechanism of producing a tissue adhesive effect consists in that the monomer undergoes anionic polymerization to form polycyanoacrylate, which adheres to tissues via chemical bonds. The monomer polymerization will release heat, and the monomer has certain biological toxicity, which may burn tissues and endanger the body safety. Consequently, they are somewhat restricted in clinical applications. Besides, when the tissue wound surface is in a wet state, the α-cyanoacrylate-based products cannot be polymerized to achieve tissue adherence based on their polymerization principle.

In Surgiflo absorbable fluid gelatin products, a certain dose of glycerol is added as a thickener. Such kind of semi-solid materials is diluted with normal saline into a slurry form before use and then injected into tissues. On the one hand, the materials generate a tissue adhesive effect by means of hydrogen bonding, etc.; on the other hand, the gelatin component can trigger the mechanism of the cascade reaction of platelet recruitment and body coagulation to play a hemostatic role.

For two modified polyethylene glycol-based tissue adhesive material of Coseal, the two kinds of powder are dissolved separately in normal saline or in a phosphate buffer solution before use and then injected into tissues with their specific injection devices to form solid adhesive hydrogels *in situ* in the tissues, which creates a tissue sealing effect as a means of mechanical closure.

Since these products are usually required to be prepared in advance before use and then topically injected or filled into the damaged areas with the dissolved materials, the products cannot be used directly in scenarios such as wound hemostasis. There is no doubt that this process increases the preoperative preparation time and extends the operative treatment time. Furthermore, it is usually necessary to cooperate with specific medical apparatus and instruments to enable these products to be formulated and properly used, which poses certain difficulties and obstacles to the procedures of clinical emergency operations.

Moreover, these commercially available tissue sealing materials have very low tissue adhesiveness, and when applied *in vivo* and *in vitro,* they may detach from the application sites due to not high adhesiveness.

Therefore, there still remains a need to develop a ready-to-use tissue sealing material to provide greater convenience for operative procedures and treatment and a need to provide a tissue sealing material with stronger tissue adhesiveness.

### SUMMARY

To address the problems about low operability and inadequate convenience and portability of current tissue sealing materials and to further improve the tissue adhesiveness of tissue sealing materials, the present disclosure provides a PEG bicomponent absorbable biological patch with self-adhesiveness, which can be firmly adhered to the tissue surface based on the specific tissue adherence mechanism, and whose advantage lies particularly in direct use for adhering tissues without extra preparation. Additionally, the biological patch has good biocompatibility, has biodegradability, and can be used in common clinical scenarios such as tissue adherence, wound hemostasis, and low-pressure leakage of body fluids.

In the present disclosure, the meaning of "biodegradable" is as same as that of "bioabsorbable" or "absorbable", all of which have a known and generic meaning in the art. That is, the patch materials of the present disclosure can fully exert their functions in the body for a period of time; then they start to degrade and lose their original functions after a period of time, and their degradation products are absorbed or excreted after metabolism, without residues in the body.

The first aspect of the present disclosure provides a PEG bicomponent self-adhesive absorbable biological patch.

According to the present disclosure, the PEG bicomponent self-adhesive absorbable biological patch comprises a substrate layer and an adhesive coating located on the substrate layer.

According to the present disclosure, the substrate layer is a biological material, which is biodegradable, preferably a biological material substantially containing a collagen protein. The collagen protein may be naturally sourced, synthesized, modified or crosslinked, which includes, but is not limited to, submucosa, dermis, pericardium, collagen, gelatin and the like. In some embodiments of the present disclosure, the substrate layer is small intestinal submucosa, preferably decellularized small intestinal submucosa. In some embodiments of the present disclosure, the substrate layer is dermis, preferably decellularized dermis. In some embodiments of the present disclosure, the substrate layer is pericardium, preferably decellularized pericardium. The submucosa (such as small intestinal submucosa), dermis, and pericardium and the like are preferably derived from mammals, for example, pigs, cattle, sheep, dogs, cats and the like. In one embodiment of the present disclosure, the substrate layer is decellularized porcine small intestinal submucosa.

According to the present disclosure, the substrate layer has a thickness of 0.01 mm to 1 mm, generally 0.05 mm to 0.5 mm, preferably 0.08 mm to 0.3 mm, *e.g.*, 0.08 mm, 0.09 mm, 0.1 mm, 0.11 mm, 0.12 mm, 0.14 mm, 0.16 mm, 0.18 mm, 0.2 mm, 0.24 mm, or 0.26 mm.

The substrate layer may be a homogeneous layer or may be in the form of a laminate formed by two or more separable layers. When the substrate layer is in the form of a laminate, the composition of each of the separable layers may or may not be the same. For example, the substrate layer is in the form of a laminate formed by several layers of small intestinal submucosa, and the small intestinal submucosa is preferably decellularized small intestinal submucosa; alternatively, the outermost separable layer of the substrate layer may be decellularized small intestinal submucosa, and the intermediate separable layer may be collagen. When the substrate layer is in the form of a laminate, the thickness of each of the separable layers may or may not be the same. For example, the thickness of the outermost separable layer of the substrate layer is greater than that of the intermediate separable layer. In one embodiment of the present disclosure, the substrate layer is composed of 2 to 9 layers of the decellularized small intestinal submucosa, for example, composed of 2 layers, 3 layers, 4 layers, 5 layers, 6 layers, 7 layers, 8 layers or 9 layers of the decellularized small intestinal submucosa.

According to the present disclosure, the adhesive coating comprises polyethylene glycol succinimidyl ester and polyethylene glycol amine. The adhesive coating is also biodegradable.

According to the present disclosure, the adhesive coating is in a dry state before use, and its moisture content is preferably less than or equal to 10.0 wt.%, further preferably less than or equal to 5.0 wt.%, more preferably less than or equal to 1.0 wt.%, based on the total weight of the adhesive coating.

According to the present disclosure, in the adhesive coating, polyethylene glycol succinimidyl ester and polyethylene glycol amine may be present in the same layer in a mixed form, or may be present in the adhesive coating in a separable form of a polyethylene glycol succinimidyl ester layer and a polyethylene glycol amine layer. Whichever form they are present, the weight ratio of polyethylene glycol succinimidyl ester to polyethylene glycol amine in the whole adhesive coating is 50:1 to 1:1, preferably 10:1 to 1:1. In the whole adhesive coating, the amount of polyethylene glycol succinimidyl ester is 0.5 to 3 mg/cm², preferably 0.6 to 2.0 mg/cm²; and the amount of polyethylene glycol amine is 0.02 to 2.0 mg/cm², preferably 0.1 to 1.5 mg/cm². In some embodiments of the present disclosure, in the whole adhesive coating, the amount of polyethylene glycol succinimidyl ester is 0.8 to 1.2 mg/cm²; and the amount of polyethylene glycol amine is 0.2 to 0.8 mg/cm².

According to the present disclosure, the polyethylene glycol succinimidyl ester may be single-armed or multi-armed, such as double-armed, four-armed, six-armed, eight-armed, or ten-armed. Its number-average molecular weight may be 1,000 to 100,000, for example, 2,000, 3,000, 4,000, 5,000, 6,000, 7,000, 8,000, 9,000, 10,000, 11,000, 12,000, 13,000, 14,000, 15,000, 16,000, 17,000, 18,000, 19,000, 20,000, 30,000, 40,000, 50,000, 60,000, 70,000, 80,000, 90,000, or 95,000. In some embodiments of the present disclosure, its number-average molecular weight is 1,000 to 50,000. The polyethylene glycol succinimidyl ester is polyethylene glycol with the succinimidyl ester structure including, but not limited to, polyethylene glycol succinimidyl glutaric ester, polyethylene glycol succinimidyl oxalic ester, polyethylene glycol succinimidyl malonic ester, polyethylene glycol succinimidyl succinic ester, and polyethylene glycol succinimidyl adipic ester. In some embodiments of the present disclosure, the polyethylene glycol succinimidyl ester is polyethylene glycol succinimidyl glutarate ester. In a specific embodiment of the present disclosure, the polyethylene glycol succinimidyl ester is four-arm polyethylene glycol succinimidyl glutarate ester.

According to the present disclosure, the polyethylene glycol amine may be single-armed or multi-armed, such as double-armed, four-armed, six-armed, eight-armed, or ten-armed. Its number-average molecular weight may be 1,000 to 100,000, for example, 2,000, 3,000, 4,000, 5,000, 6,000, 7,000, 8,000, 9,000, 10,000, 11,000, 12,000, 13,000, 14,000, 15,000, 16,000, 17,000, 18,000, 19,000, 20,000, 30,000, 40,000, 50,000, 60,000, 70,000, 80,000, 90,000, or 95,000. In some embodiments of the present disclosure, its number-average molecular weight is 1,000 to 50,000. In a specific embodiment of the present disclosure, the polyethylene glycol amine is four-arm polyethylene glycol amine.

According to the present disclosure, the adhesive coating may be continuous or discontinuous. Said "discontinuous" in the present disclosure indicates that the adhesive coating may be reticular or contains voids. From the standpoint of providing stronger tissue adhesiveness, it is preferred that the adhesive coating is continuous.

According to the present disclosure, the adhesive coating may further contain an additive. The additive includes, but is not limited to, chelating agents, preservatives, active pharmaceuticals and the like. The chelating agents may be reagents for increasing the degree of crosslinking of the polyethylene glycol bicomponent, which include, but are not limited to, tannic acid, trilysine and the like. The preservatives may be benzoic acid, sodium benzoate, sorbic acid, potassium sorbate, and the like. The active pharmaceuticals may be procoagulants (such as fibrinogen, thrombin or thrombin precursors), antibiotics such as antibacterial or antifungal antibiotics, growth factors, analgesics, and the like.

According to the present disclosure, when the adhesive coating is in the form of a laminate formed by a plurality of separable layers, the composition of each of the separable layers may or may not be the same. For example, the adhesive coating is formed by a layer of polyethylene glycol succinimidyl ester, a layer of polyethylene glycol amine, a layer of polyethylene glycol succinimidyl ester, and a layer of polyethylene glycol amine in an alternate manner. Alternatively, the separable layer as the outermost layer of the adhesive coating is composed of a mixture of polyethylene glycol succinimidyl ester and polyethylene glycol amine, and the intermediate separable layer may contain only polyethylene glycol succinimidyl ester or polyethylene glycol amine. When the adhesive coating is in the form of a laminate formed by a plurality of separable layers, the thickness of each of the separable layers may or may not be the same. For example, the separable layer as the outermost layer of the adhesive coating may be thicker than the intermediate separable layer. In some embodiments of the present disclosure, the adhesive coating is composed of, in the direction from the surface in contact with the substrate layer to the free surface, a polyethylene glycol succinimidyl ester layer and a polyethylene glycol amine layer laminated in this order. In some embodiments of the present disclosure, the adhesive coating is composed of, in the direction from the surface in contact with the substrate layer to the free surface, a polyethylene glycol amine layer and a polyethylene glycol succinimidyl ester layer laminated in this order.

The term "patch" means an article whose thickness is much less than other dimensions thereof. Such an article in the present disclosure may also be expressed as sticking-plaster or film or sheet or the like.

The present disclosure further provides a combination product of a PEG bicomponent self-adhesive absorbable biological patch, the combination product comprising a substrate layer and an adhesive coating, which are packaged separately.

The substrate layer is as defined and described as the above-mentioned "substrate layer".

Likewise, the adhesive coating is as defined and described as the above-mentioned "adhesive coating".

When the combination product is in use, the substrate layer and the adhesive coating that are packaged separately are taken out from packages. After the surface of the substrate layer to which the adhesive coating is to be adhered is infiltrated with an aqueous solution, the adhesive coating is adhered to this surface, and the free surface of the adhesive coating is then adhered to the tissue wound surface.

According to the present disclosure, the aqueous solution is a solution with water as a solvent system, and has a pH of preferably 6.9 to 7.5, most preferably 7.0 to 7.4, which includes, but is not limited to, water, normal saline, physiologically acceptable buffer solutions such as PBS, etc.

The present disclosure further provides a method of preparing a PEG bicomponent self-adhesive absorbable biological patch.

According to the present disclosure, the method comprises forming an adhesive coating on a substrate layer.

According to the present disclosure, the method of forming the adhesive coating may be the method of coating a polyethylene glycol succinimidyl ester solution and a polyethylene glycol amine solution on the substrate layer. The polyethylene glycol succinimidyl ester solution has a concentration of 5.0 to 30.0 wt.%, preferably has a concentration of 10.0 to 20.0 wt.%; the polyethylene glycol amine solution has a concentration of 0.2 to 20.0 wt.%, preferably has a concentration of 1.0 to 15.0 wt.%; and the solvents of both solutions are an aqueous solution. In some embodiments of the present disclosure, the polyethylene glycol succinimidyl ester solution has a concentration of 8.0 to 12.0 wt.%, and the polyethylene glycol amine solution has a concentration of 2.0 to 8.0 wt.%.

According to the present disclosure, the coating may be a coating method known in the art, and may be manual coating or machine coating.

According to the present disclosure, the polyethylene glycol succinimidyl ester solution and the polyethylene glycol amine solution may be coated simultaneously, for example, they are mixed and then coated; or may be alternately coated in turn, for example, the polyethylene glycol succinimidyl ester solution is coated before the polyethylene glycol amine solution is coated, or the polyethylene glycol amine solution is coated before the polyethylene glycol succinimidyl ester solution is coated.

According to the present disclosure, when two kinds of polyethylene glycol solutions are alternately coated in turn, after a first polyethylene glycol solution is coated, the first polyethylene glycol solution may or may not be subjected to dehydration treatment before coating a second polyethylene glycol solution, that is, the second polyethylene glycol may be coated when the first polyethylene glycol is in a dry or liquid state. From the standpoint of forming separable layers, it is preferred that the second polyethylene glycol is coated when the first polyethylene glycol is in a dry state. Said drying may include freeze-drying or air drying (*e.g.*, vacuum drying or heat drying), and include removal of volatile components from the liquid. In one embodiment of the present disclosure, freeze-drying is used.

After completion of all coating, the resulting self-adhesive absorbable biological patch is subjected to dehydration treatment. The dehydration treatment may be carried out by a dehydration method known in the art, including, but not limited to, freeze-drying, vacuum drying, heat drying and the like. In one embodiment of the present disclosure, freeze-drying is used.

According to the present disclosure, in the case of containing an additive, the additive may be added to the adhesive coating system by means of being added to a mixture of the two kinds of polyethylene glycol, or to either of the two kinds of polyethylene glycol, or being coated after the two kinds of polyethylene glycol are coated. In one embodiment of the present disclosure, the additive is added to the adhesive coating system by means of being added to a polyethylene glycol amine solution. In another embodiment of the present disclosure, the additive is added to the adhesive coating system by means of being coated after the two kinds of polyethylene glycol are coated.

When a combination product of the PEG bicomponent self-adhesive absorbable biological patch is prepared, the above-mentioned preparation method may be adopted with the only difference in nonuse of the substrate layer, while other non-adhesive material is used as a substrate and the adhesive coating is formed on the substrate.

The present disclosure further provides a precursor solution composition for forming the adhesive coating, the composition comprising a polyethylene glycol succinimidyl ester solution and a polyethylene glycol amine solution that are present independently, wherein the polyethylene glycol succinimidyl ester solution has a concentration of 5.0 to 30.0 wt.%, preferably has a concentration of 10.0 to 20.0 wt.%; the polyethylene glycol amine solution has a concentration of 0.2 to 20.0 wt.%, preferably has a concentration of 1.0 to 15.0 wt.%; and the solvents of both solutions are an aqueous solution. In some embodiments of the present disclosure, the polyethylene glycol succinimidyl ester solution has a concentration of 8.0 to 12.0 wt.%, and the polyethylene glycol amine solution has a concentration of 2.0 to 8.0 wt.%.

According to the present disclosure, the precursor solution composition may further comprise an additive solution or solid additive that is present independently.

The present disclosure further provides a kit, comprising an individually packaged substrate layer, and an individually packaged precursor solution composition for forming an adhesive coating. The precursor solution composition is as described previously.

When in use, the substrate layer and the precursor solution composition are taken out from the packages, and prepared into a usable patch by the method of preparing a self-adhesive absorbable biological patch described above in the present disclosure.

The present disclosure further provides a therapeutic agent delivery device for sealing a tissue surface and releasing one or more therapeutic agents to a target site, the device comprising: (i) a PEG bicomponent self-adhesive absorbable biological patch, and (ii) a plaster loaded with one or more therapeutic agents, wherein the area of the plaster is smaller than the area of the PEG bicomponent self-adhesive absorbable biological patch, and the plaster is located on a free surface of an adhesive coating of the PEG bicomponent self-adhesive absorbable biological patch. The PEG bicomponent self-adhesive absorbable biological patch is as described previously in the present disclosure.

The present disclosure further provides use of the PEG bicomponent self-adhesive absorbable biological patch in the preparation of a tissue sealing material.

The present disclosure further provides a method of sealing a tissue wound or wound surface, the method comprising coating a PEG bicomponent self-adhesive absorbable biological patch according to the present disclosure to a tissue wound or wound surface in need of being sealed, with adhering a free surface of an adhesive coating of the biological patch to the tissue wound or wound surface in need of being sealed.

The present disclosure further provides a method of releasing one or more therapeutic agents to a target site, comprising: providing a therapeutic agent delivery device as described above in the present disclosure, and placing a free surface of an adhesive coating of the PEG bicomponent self-adhesive absorbable biological patch into contact with a tissue surface.

The surface of the tissue to be sealed in the present disclosure may be dry or wet. For a dry tissue surface, the surface may be wetted with an aqueous solution before coating the self-adhesive absorbable biological patch. For a wet tissue surface, the self-adhesive absorbable biological patch may be coated directly thereon or the surface may be further wetted with an aqueous solution before coating the self-adhesive absorbable biological patch.

For the PEG bicomponent self-adhesive absorbable biological patch of the present application, by forming a bicomponent adhesive coating from polyethylene glycol succinimidyl ester and polyethylene glycol amine and reducing the initial reaction rate, the two components further react with each other after the bicomponent adhesive coating is bonded to the wound surface to form an adhesive hydrogel on the substrate layer by means of polymerization and crosslinking, and to make the tissue and the substrate layer bonded adequately to each other through its self-adhesiveness. After the self-adhesive absorbable biological patch is in contact with a wet tissue, its tissue viscosity is derived from acting forces such as static electricity, amide bonds and the like between the chemical groups such as free amino groups, succinimidyl esters and the like in the adhesive coating and the free groups on the tissue surface, thereby forming tissue adherence.

By selecting the content ratio of the PEG bicomponent, the present disclosure ensures that the PEG bicomponent is distributed uniformly in the surface of the substrate layer without any obvious interface while forming a hydrogel with strong adhesiveness, and the mechanical properties of the resulting hydrogel are adapted to those of the substrate layer, so that the tissue compliance and mechanical properties of the resulting self-adhesive absorbable biological patch are adapted to those of the body tissue. Such a combination not only addresses the problems of insufficient mechanical strength, stress shielding, and vulnerability when the PEG bicomponent adhesive coating is used alone, but also addresses the problem of non-selectivity to the object to be adhered when the PEG bicomponent adhesive coating is used alone, such that the substrate layer plays a role of overcoming the stress shielding of the PEG bicomponent adhesive coating on the one hand, and exerts the closure effect of the adhesive coating on the other hand; as a result, when applied to adherence of *in vivo* tissues, it will not cause adhesion to non-target adhesive tissues.

The PEG bicomponent self-adhesive absorbable biological patch of the present disclosure has a degree of flexibility and can withstand the tensile strain within 10% of itself, and its Young's modulus can be as high as 255.22 MPa, which satisfies the dynamic mechanical microenvironment *in vivo*, so that the biological patch can adhere tightly to the tissue surface, and even if the tissue is deformed due to the pulsation or twisting, the biological patch can still maintain the tight adherence. In accordance with the ASTM F2392 burst strength test standard for tissue adhesives, the burst strength of the self-adhesive patch may be as high as 32 kPa, which is significantly higher than that of other commercialized tissue adhesive products. Since the normal arterial blood pressure and pulmonary physiological pressure of human body are in the range of 10 to 16 kPa and 2.67 kPa respectively, the adhesive strength of the adhesive material of the present disclosure is above this range, indicating that it can be used for hemostasis and lung air leakage sealing.

In addition, the PEG bicomponent self-adhesive absorbable biological patch of the present disclosure dispenses with a complicated preparation process, and thus fulfills the purpose of ready-to-use tissue adhesive materials; it has strong adhesiveness, which can avoid operative suture, has little damage to the wound surface, and avoids the hidden dangers of stress concentration, inflammatory response and capillary damage caused by suture needles to tissues; the PEG bicomponent self-adhesive absorbable biological patch is bio-safety and degradable.

The performance comparisons between the PEG bicomponent self-adhesive absorbable biological patch of the present disclosure and other commercialized tissue adhesives are listed in the tables below.

**Table 1 Comparison of tissue adhesive strength between PEG bicomponent self-adhesive absorbable biological patch and commercialized tissue adhesive materials**

| Name | Tissue adhesive strength (kPa) |
|---|---|
| Self-adhesive absorbable biological patch | ~33.4 |
| TissuePatch | ~20 |
| Hemopatch | ~1 |

**Table 2 Comparison of cytocompatibility between PEG bicomponent self-adhesive absorbable biological patch and commercialized tissue adhesive materials**

| Name | Cell Activity (%) |
|---|---|
| Self-adhesive absorbable biological patch | ~90 |
| α-Cyanoacrylate | ~50 |
| BioGlue | ~60 |

**Table 3 Comparison of biodegradability between PEG bicomponent self-adhesive absorbable biological patch and commercialized tissue adhesive materials**

| Name | Degradation Cycle (Day) |
|---|---|
| Self-adhesive absorbable biological patch | 30 to 90 |
| TissuePatch | ~40 |
| Hemopatch | ~30 |
| α-Cyanoacrylate | Non-degradable |
| BioGlue | Non-degradable |

Therapeutic use of the PEG bicomponent self-adhesive absorbable biological patch of the present disclosure

The biological patch of the present disclosure is applicable to both the internal surfaces and external surfaces of the body, that is, it may be applied topically to the exterior of the body (*e.g.,* applied to the skin), or applied topically to the internal surfaces, such as surfaces of internal organs exposed during operation or trauma, and the operation includes both conventional operations and minimally invasive operations.

The biological patches of the present disclosure are particularly suitable for the internal surfaces of the body.

The biological patches of the present disclosure are particularly suitable for operative applications in the following areas: thoracic/cardiovascular, otolaryngology, urinary system, oral/maxillofacial, plastic surgery, nervous system, gastrointestinal system, ophthalmology, gynecology/obstetrics. Exemplary uses are as follows:

### Skin Closure

Biological patches may be applied topically to promote wound closure (as an alternative to sutures), which can reduce scars. Therefore, in minor operations (*e.g.*, in accidents and first-aids), the self-adhesiveness of the biological patches, if used, allows for ease of their rapid application.

### Wound Healing

The degradability of the biological patch means that it can support and promote wound healing. Once the biological patch starts to degrade, fibroblasts will migrate into the extracellular matrix and deposit therein. Therefore, the biological patch may be used as a dressing for the internal or external surface. Besides, factors known to promote skin cell proliferation, such as growth factors and cAMP, may be added to the biological patch to promote healing. An anti-infective drug or the like may also be added to the biological patch to make it particularly useful for treating burns.

### Hernia Repair

In herniorrhaphy, biological patches may be used to provide reinforcement. The self-adhesive connection overcomes the potential problems faced with conventional surgical reinforcement patch products, such as the need to cooperate with suturing or stapling. Biological patches for use in herniorrhaphy may be designed to have short-term or long-term durability depending on the requirements for tissue repair. Of course, the biological patches of the present disclosure can withstand stapling.

### Anastomosis

Self-adhesive patches provide a means to quickly seal and connect tubular structures such as blood vessels, vascular grafts, bladder grafts, and gastrointestinal tracts, and prevent them from leaking.

### Large Tissue Area Sealing

Good sealing and handling properties of the biological patch, together with its self-adhesiveness and ability to cover a large surface area, mean that it is particularly useful for sealing excised tissue surfaces, especially those (*e.g.,* liver, spleen, and kidney) where diffuse bleeding occurs. The biological patch may also provide a desirable support matrix for tissue repair at these locations. The biological patch may also be used to limit the leakage of cerebrospinal fluid after neurosurgery.

### Air Leakage Sealing

The high tensile strength and good inherent elasticity of the biological patch (after hydration and reaction with active functional groups of the tissue) make it particularly suitable for sealing air leakage in the lung, especially after pneumonectomy. Moreover, after sealing, the biological patch provides a desirable support matrix for tissue repair at these locations.

### Hemostasis

The biological patch may be applied to a bleeding area as a physical barrier. Tissue active materials in the biological patch may immobilize proteins to thereby promote hemostasis.

### Administration of Therapeutic Agents

Drugs and other therapeutic agents (including biologically active agents such as growth factors, and even cells and cellular components) may be added to the solution used for forming the adhesive coating component, or covalently bonded to the component before the component is used to manufacture a biological patch.

### Postoperative Adhesion Prevention

Postoperative adhesion is formation of undesirable connective tissues between adjacent tissues, which is prone to serious postoperative complications. For example, in intestinal operations, the postoperative adhesion may cause intestinal entanglement, which leads to a further operation. Applying the biological patch with self-adhesiveness in the present disclosure to the tissue exposed during operation can effectively prevent postoperative adhesion between this tissue and adjacent tissues.

### Minimally Invasive Operation

The adoption of minimally invasive techniques to collect tissue samples through biopsy, insert instruments, deliver therapeutic agents, and implement operative approaches has become an alternative to conventional "open" operations. The biological patch is shaped or manufactured into an appropriate size and configuration, and introduced into the body through a specially designed available minimally invasive operative instrument and trocar system, and its self-adhesiveness is utilized to significantly reduce the technical difficulties related to manipulation, closure, and repair of tissues.

The biological materials as the substrate layer are all biological materials known in the art, or may be prepared by the methods known in the art.

For example, SIS, dermis, and pericardium are all known in the art. It is also known in the art to decellularize them to obtain decellularized extracellular matrices of the corresponding tissues. Decellularized SIS, decellularized dermis, and decellularized pericardium have been commercially available, or may also be prepared by methods known in the art.

For example, in some embodiments of the present disclosure, SIS or dermis or pericardium is treated with ultrasound to obtain decellularized SIS, decellularized dermis or decellularized pericardium. In one embodiment of the present disclosure, the frequency of the ultrasonic treatment is 20 to 80 KHz. In another embodiment of the present disclosure, the ultrasonic treatment is multi-frequency ultrasonic treatment, and the low-frequency ultrasonic treatment is used before the high-frequency ultrasonic treatment. Preferably, the low-frequency range is from 20 to 40 KHz, and the high-frequency range is from 60 to 90 KHz, where the low-frequency treatment is carried out for 5 to 40 min, and the high-frequency treatment is carried out for 5 to 40 min.

In some embodiments of the present disclosure, the SIS or dermis or pericardium is treated with a solution containing trypsin and EDTA or a salt thereof to obtain decellularized SIS, decellularized dermis or decellularized pericardium. Preferably, the concentration of trypsin in the solution is 0.01 to 0.2% by mass, preferably 0.02 to 0.05% by mass, and the concentration of EDTA or a salt thereof (such as EDTA-2Na or EDTA-4Na) is 0.1 to 1 mmol/L, preferably 0.4 to 0.8 mmol/L; the pH value of the solution is 7.0 to 8.0, preferably 7.2 to 7.5.

In some embodiments of the present disclosure, multi-frequency ultrasound and a solution containing trypsin and EDTA or a salt thereof are used in combination to treat the SIS or dermis or pericardium to obtain decellularized SIS, decellularized dermis or decellularized pericardium.

In some embodiments of the present disclosure, decellularized SIS, decellularized dermis, decellularized pericardium or the like may be prepared by the preparation methods disclosed in Chinese Patent No. CN103272278B or CN107007886B, the entire contents of which are incorporated herein.

The collagen used in the present disclosure may be derived from any collagen suitable for gel formation, including substances from liquid, slurry, fibrous or powdery collagen materials that can be processed into porous or fibrous matrices. To improve the gel-forming ability or solubility, the collagen may be (partially) hydrolyzed or modified as long as a stable substrate layer is formed when drying.

Natural gelatin is inexpensive and available in large quantities, and can be gained from many sources. Gelatin is a hydrolysate of collagen. Convenient animal sources of gelatin and collagen include chicken, turkey, cattle, pig, horse or human sources. Collagen may also be artificial collagen or recombinant collagen. Preferably, the gelatin is crosslinked to prevent complete dissolution. Crosslinking can be achieved by incomplete hydrolysis of collagen or by chemical crosslinking using a crosslinking agent such as formaldehyde or divalent aldehyde.

### Definitions of Terms:

Unless otherwise defined, all technical and scientific terms used herein have the same meanings as commonly understood by one of ordinary skill in the art. **In** case of any contradiction, the definitions provided in the present application shall prevail. Trade name, when used herein, is intended to refer to its corresponding merchandise or its active ingredient. Patents, published patent applications, and publications cited herein are all incorporated herein by reference.

The term "comprising", "including", "having", "containing" or "involving" and other variants thereof used herein are intended to be inclusive or open-ended, and does not exclude other unrecited elements or methods and steps. It should be appreciated to a person skilled in the art that the above term such as "comprising" encompasses the meaning of "consisting of ... ".

The terms "selected from the group consisting of ...", "preferably ...", and "more preferably ..." refer to independent selection from one or more elements in the group listed thereafter, and may include a combination of two or more elements. In the present disclosure, one of the elements in the group listed thereafter is preferred.

The term "optional", "optionally", or "optionally present" means that the event or situation described subsequently may, but does not necessarily, occur, and the description includes the case where the event or situation occurs and the case where the event or situation does not occur.

In the present disclosure, unless otherwise specified, the "molecular weight" of polyethylene glycol derivatives refers always to the weight-average molecular weight of polyethylene glycol derivatives.

In the present disclosure, the term "may" is used to indicate two meanings of performing and not performing certain processing.

In the present disclosure, the numerical range represented by "numerical value A to numerical value B" is used to indicate the range including the endpoint values A and B.

In the present disclosure, "water" includes any usable water such as deionized water, distilled water, ion exchange water, double distilled water, high purity water, and pure water.

In the present disclosure, the free surface of the adhesive coating refers to the surface of the adhesive coating in contact with the tissue, which is opposite to the contact surface between the adhesive coating and the substrate layer.

### BRIEF DESCRIPTION OF THE DRAWINGS

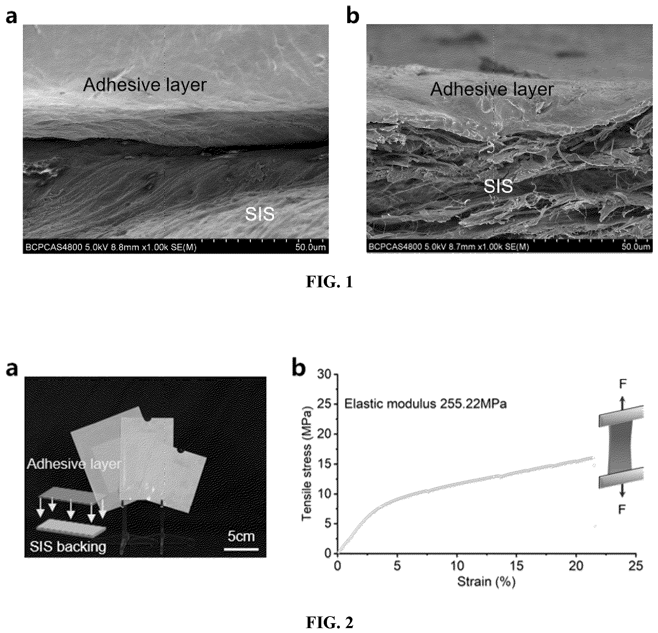

FIG. 1 (a) is an SEM image of the PEG bicomponent after rapid gelation on the surface of the SIS substrate layer, showing that an obvious interface occurs between the adhesive layer (*i.e.,* the adhesive coating) and the substrate layer; (b) is an SEM image of the PEG bicomponent after slow gelation on the surface of the SIS substrate layer, showing that the adhesive layer (*i.e.,* the adhesive coating) and the substrate layer are bonded tightly to each other, without an obvious interface therebetween.
FIG. 2 (a) is the appearance of a PEG bicomponent self-adhesive absorbable biological patch: the decellularized extracellular matrix of porcine small intestinal submucosa (SIS) is used as a substrate layer, and a polyethylene glycol bicomponent adhesive coating is coated on the surface of the substrate layer, and freeze-dried to show the appearance; (b) is a stress-strain curve of the self-adhesive absorbable biological patch, which can withstand the tensile strain within 10% of itself and has a Young's modulus of up to 255.22 MPa.

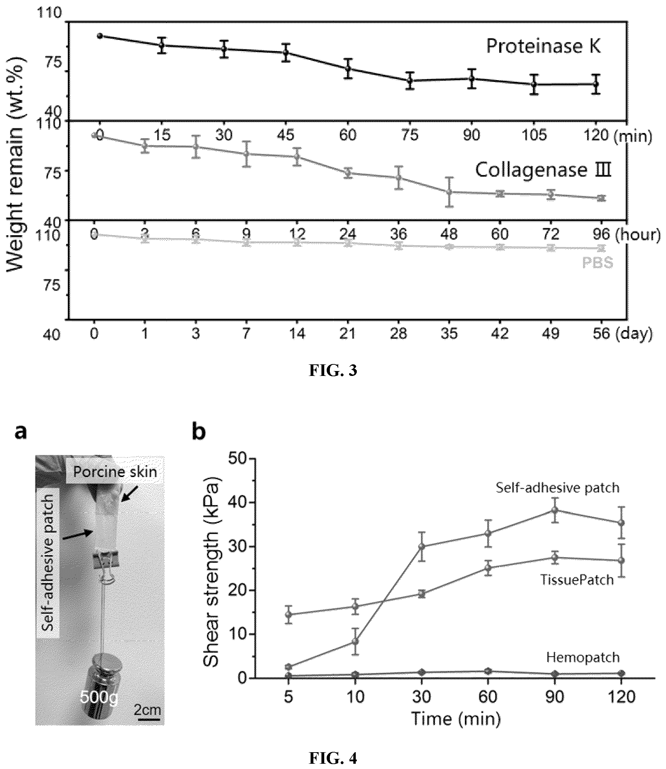

FIG. 3 shows a statistic diagram for *in vitro* enzymolysis of a PEG bicomponent self-adhesive absorbable biological patch. The materials are soaked in an aqueous proteinase K solution, an aqueous collagenase solution, and a PBS solution respectively, and the weights of the biological patches are measured at the specified time.
FIG. 4 (a) is a schematic diagram for shear tensile of a PEG bicomponent self-adhesive absorbable biological patch after adhered to the pigskin for 30 min, which can bear the weight of 500g weights; (b) shows the relationship between the adhesive strength and time after the biological patch is quantitatively brought into contact with the tissue, where the adhesive strength of the biological patch increases gradually with the extension of its contact time with the tissue, and 30 min later, its adhesive strength exceeds that of other commercialized tissue adhesive materials.

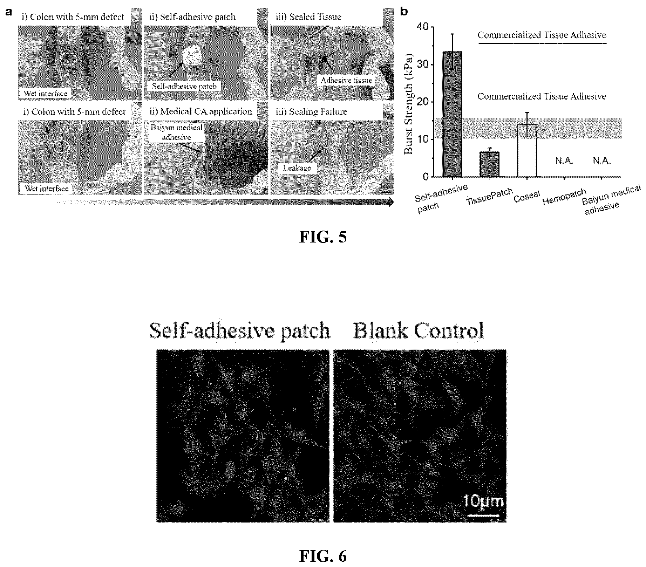

FIG. 5 (a) is an *in vitro* experiment on sealing of colonic defects, showing that the PEG bicomponent self-adhesive absorbable biological patch is useful for tissue sealing adherence in a wet state, while the Baiyun medical adhesive (with the component of n-octyl cyanoacrylate)
cannot be adhered to a wet surface; (b) is a contrast image of the burst strength between the PEG bicomponent self-adhesive absorbable biological patch and other commercialized tissue adhesives.

FIG. 6 shows staining diagrams of the cell skeleton of mouse fibroblast L929 of the leach liquor of a PEG bicomponent self-adhesive absorbable biological patch and a blank control.

### DETAILED DESCRIPTION

The technical solution of the present disclosure will be further explained in detail below with reference to specific examples. It should be appreciated that the following examples are only intended to schematically illustrate and explain the present disclosure and shall not be construed as limitations on the scope of protection for the present disclosure. Any technology implemented based on the above contents of the present disclosure is covered in the scope sought to be protected by the present disclosure.

Unless otherwise stated, the raw materials and reagents used in the examples below are all commercially available or may be prepared by known methods.

### Example 1: Preparation of PEG bicomponent self-adhesive absorbable biological patch

Four-arm polyethylene glycol succinimidyl glutarate ester (PEG-SG, Xiamen Nanopeg Company, the number-average molecular weight: 10,000, also used in the following examples) and four-arm polyethylene glycol amine (PEG-NH₂, Xiamen Nanopeg Company, the number-average molecular weight: 10,000, also used in the following examples) were dissolved in purified water respectively, and formulated into solutions at different concentrations of 5.0 to 60.0 wt.% and 0.2 to 60.0 wt.% respectively for use in the preparation of a self-adhesive absorbable biological patch.

Tissue shear tensile viscosity test was one of the standard methods for testing the adhesive strength of tissue adhesives, which was used to determine the average load per unit bond area (Pa) when two bonded adherends were gradually separated from each other. The decellularized extracellular matrix of 100 cm² porcine small intestinal submucosa (SIS) (the product of Beijing Biosis Healing Biological Technology Co., Ltd., 0.17 mm thick, composed of 4 layers of decellularized small intestinal submucosa) was taken as a substrate layer. A PEG-SG solution was coated first on the substrate layer, and after drying, a PEG-NH₂ solution was then coated and subjected to freeze-drying treatment to form a self-adhesive absorbable biological patch. With reference to ASTM F2255, after the self-adhesive absorbable biological patch and pigskin were bonded and interacted for 30 min, the shear tensile strength test was carried out. The results were as follows. In addition, the prepared self-adhesive absorbable biological patch was observed with SEM to learn about the microstructure between the substrate layer and the adhesive coating. It had been found in the experiment that when the PEG-SG concentration was higher than 30% and the PEG-NH₂ concentration was higher than 20%, an obvious interface would occur between the substrate layer and the adhesive coating.

| Test Sample No. | PEG-SG Concentration (wt.%) | PEG-SG Amount (mg/cm²) | PEG-NH₂ Concentration (wt.%) | PEG-NH₂ Amount (mg/cm²) | Shear Strength (kPa) |
|---|---|---|---|---|---|
| 1 | 5 | 0.5 | 0.2 | 0.02 | 3.92 |
| 2 | 5 | 0.5 | 20 | 2 | 1.90 |
| 3 | 10 | 1 | 0.2 | 0.02 | 5.56 |
| 4 | 10 | 1 | 1 | 0.1 | 3.45 |
| 5 | 10 | 1 | 2 | 0.2 | 17.07 |
| 6 | 10 | 1 | 5 | 0.5 | 22.3 |
| 7 | 20 | 2 | 1 | 0.1 | 8.54 |
| 8 | 20 | 2 | 2 | 0.2 | 12.43 |
| 9 | 20 | 2 | 5 | 0.5 | 13.17 |
| 10 | 30 | 3 | 1 | 0.1 | 2.26 |
| 11 | 30 | 3 | 2 | 0.2 | 16.57 |
| 12 | 30 | 3 | 5 | 0.5 | 10.7 |
| 13 | 40 | 4 | 1 | 0.1 | 6.77 |
| 14 | 40 | 4 | 2 | 0.2 | 8.33 |
| 15 | 40 | 4 | 5 | 0.5 | 9.70 |

### Example 2: Preparation of PEG bicomponent self-adhesive absorbable biological patch

SIS (the product of Beijing Biosis Healing Biological Technology Co., Ltd., 0.17 mm thick, composed of 4 layers of decellularized small intestinal submucosa) was used as a substrate layer. 1 mL of 10.0 wt.% PEG-SG solution was coated first, and after drying, 1 mL of 5.0 wt.% PEG-NH₂ solution was then coated on 100 cm² SIS, frozen at -20°C, and subjected to freeze-drying treatment to obtain a self-adhesive absorbable biological patch.

### Example 3: Preparation of PEG bicomponent self-adhesive absorbable biological patch

SIS (the product of Beijing Biosis Healing Biological Technology Co., Ltd., 0.17 mm thick, composed of 4 layers of decellularized small intestinal submucosa) was taken as a substrate layer. 1 mL of PEG-NH₂ solution at a concentration of 5.0 wt.% was coated first, and after drying, 1 mL of PEG-SG solution at a concentration of 10.0 wt.% was then coated on 100 cm² SIS, and subjected to freeze-drying treatment at -20°C to form a self-adhesive absorbable biological patch.

### Example 4: Preparation of PEG bicomponent self-adhesive absorbable biological patch

The bovine pericardium (the bovine pericardium was subjected to freeze-drying treatment after decellularization) was used as a substrate layer. 1 mL of 10.0 wt.% aqueous PEG-SG solution was measured and coated uniformly on 100 cm² bovine pericardium, and after the bovine pericardium was infiltrated for 10 min, 1 mL of 5.0 wt.% aqueous PEG-NH₂ solution was then coated uniformly, frozen at -20°C, and then subjected to freeze-drying treatment to obtain a self-adhesive absorbable biological patch.

### Experimental Example 1: Stress-Strain Experiment

The product obtained in Example 2 was used as an experimental sample. The self-adhesive absorbable biological patch was cut into a size of 2 cm × 5 cm, and installed in a universal tensile testing machine. Parameters were set as follows: the sample was stretched at the effective length of 2 cm, the width of 2 cm, the thickness of 0.17 mm, and the tensile speed of 10 mm/min at room temperature. The Young's modulus was the slope of the stress-strain curve in the elastic deformation interval. The results were as shown in FIG. 2(b). The product of Example 2 could withstand the tensile strain within 10% of itself, and the Young's modulus reached 255.22 MPa.

### Experimental Example 2: Enzymolysis Experiment

The product obtained in Example 2 was used as an experimental sample. The 2 cm × 5 cm self-adhesive absorbable biological patch was weighed, and the initial mass was recorded as W₀. Thereafter, the self-adhesive absorbable biological patches were soaked in 30 mL of 1 mg/20 mL proteinase K solution and collagenase solution respectively and in a PBS solution as a control, and incubated in a water bath shaker at 37°C and 60 rpm. The patches were taken out every fixed time, washed with deionized water, and then freeze-dried and weighed. The mass was recorded as Wₜ. Weight loss due to enzymolysis (Wₜ%) = (1 - Wₜ/W₀) × 100%. The results were as shown in FIG. 3. Under the action of proteinase K and collagenase, the weight loss due to enzymolysis of the product of Example 2 increased with the extension of the enzyme action time, whereas the weight in the PBS treatment group remained constant, indicating that the self-adhesive absorbable biological patch of the present disclosure was biodegradable.

### Experimental Example 3: Shear Strength Test

The product obtained in Example 2 was used as an experimental sample, and the other commercialized tissue adhesives (TissuePatch, Hemopatch) were used as control samples. With reference to ASTM F2255, fresh dorsal pigskin was defatted and then cut into a size of 5 cm × 2.5 cm. The samples were also cut into a size of 5 cm × 2.5 cm. Thereafter, the sample was quickly bonded to one end (with the bond area of 2.5 cm × 1 cm) of the pigskin in opposite directions, and fixed at room temperature for different time periods. The adhesive strength of each of the samples was measured after different bond time. The results were shown in FIG. 4, where a showed that the bonded interface of the product of Example 2 could bear the weight of 500 g weights, and b showed the cases where the shear strength of the samples changed over bond time. The shear strength of the product of Example 2 within the first 20 min after bonding was lower than that of TissuePatch, but with the extension of the bond time, its shear strength gradually increased to be higher than that of TissuePatch, and maintained a difference level of about 10 kPa, while Hemopatch did not show a significant measured value of the shear strength during the whole period of experimental observation.

### Experimental Example 4: Burst Experiment

The product obtained in Example 2 was used as an experimental sample. The other commercialized tissue adhesives (TissuePatch, Coseal, Hemopatch, and Baiyun medical adhesive) were used as control samples. With reference to ASTM F2392, freshly defatted porcine colon was taken and a defect with a diameter of 5 mm was created on the surface with a living perforator, and each of the above samples (2 cm × 2 cm in size) was adhered to the defect and bonded and fixed for 5 min. The colon was pressurized with a fluid perfusion device (equipped with a pressure gauge) until the fluid occlusion failed, and the maximum pressure applied was recorded as burst strength. The results were shown in FIG. 5, where a showed the real picture of the experiment, in which the self-adhesive patch of Example 2 was able to effectively bond the tissue, and leakage did not occur after perfusion with fluid, while the Baiyun medical adhesive could not effectively seal the tissue, and leakage occurred after perfusion with fluid; b showed the burst strength of each of the samples, in which the burst strength of the self-adhesive absorbable biological patch of the present disclosure was greater than 30 kPa, which was higher than ~15 kPa of Coseal and ~5 kPa of TissuePatch, whereas the Hemopatch and Baiyun medical adhesive had almost no burst strength under the test conditions, indicating that the self-adhesive absorbable biological patch of the present disclosure had excellent burst strength.

### Experimental Example 5: Cell Experiment

The product obtained in Example 2 was used as an experimental sample. The self-adhesive absorbable biological patches were soaked in cell medium at a rate of 6 cm²/mL for 24 h at 37°C. The media formed under these conditions were used to replace common media in 24-well plate inoculated with 10,000 mouse fibroblasts L929 per well. 24 h later, the cells were incubated in the dark for 2 h and 3 min using rhodamine phalloidin and DAPI fluorescent dye working solutions successively, and placed under a laser confocal microscope to observe the state of cells. Mouse fibroblasts L929 cultured in common medium under the same culture conditions were used as a blank control. The results were as shown in FIG. 6. The fluorescence image showed that there was no significant difference in the number of live cells between the self-adhesive absorbable biological patch and the blank control, indicating that this material had good cytocompatibility and could be used as a biomedical material with tissue adhesiveness.

### Comparative Example

Decellularized porcine small intestinal submucosa (SIS, the product of Beijing Biosis Healing Biological Technology Co., Ltd., 0.17 mm thick, composed of 4 layers of decellularized small intestinal submucosa) was taken as a substrate layer. Solutions of PEG-SG, PEG-NH₂, and PEG-SH (four-arm polyethylene glycol thiol, Xiamen Nanopeg Company, the number-average molecular weight: 10,000) were formulated, respectively. The method of Example 2 was adopted to prepare a self-adhesive absorbable biological patch A containing a bicomponent adhesive layer of 100 mg of PEG-SG and 50 mg of PEG-NH₂, and a self-adhesive absorbable biological patch B containing a bicomponent adhesive layer of 100 mg of PEG-SG and 50 mg of PEG-SH, respectively.

After test, the adhesive strength of the self-adhesive patch A was 34.3 kPa, and the adhesive strength of the self-adhesive patch B was 26.7 kPa, indicating that in the same cases, PEG-NH₂ as a component of the bicomponent could provide superior tissue adhesiveness to that of PEG-SH.

While the embodiments of the present disclosure have been described above, the present disclosure is not limited thereto. Any modifications, equivalent substitutions, improvements, and the like made within the spirit and principle of the present disclosure shall all be encompassed within the scope of protection for the present disclosure.

## Claims

1. A PEG bicomponent self-adhesive absorbable biological patch, comprising a substrate layer and an adhesive coating located on the substrate layer;
wherein the substrate layer is a biological material;
the adhesive coating comprises polyethylene glycol succinimidyl ester and polyethylene glycol amine, and a weight ratio of the polyethylene glycol succinimidyl ester to the polyethylene glycol amine is 50:1 to 1:1, preferably 10:1 to 1:1;
preferably, the polyethylene glycol succinimidyl ester has a number-average molecular weight of 1,000 to 100,000, and the polyethylene glycol amine has a number-average molecular weight of 1,000 to 100,000.

2. The PEG bicomponent self-adhesive absorbable biological patch according to claim 1, wherein in the adhesive coating, an amount of the polyethylene glycol succinimidyl ester is 0.5 to 3 mg/cm², preferably 0.6 to 2.0 mg/cm²; and an amount of the polyethylene glycol amine is 0.02 to 2 mg/cm², preferably 0.1 to 1.5 mg/cm²;
preferably, the adhesive coating further contains an additive.

3. The PEG bicomponent self-adhesive absorbable biological patch according to claim 1 or 2, wherein the adhesive coating is in the form of a laminate formed by a plurality of separated layers;
preferably, the adhesive coating is composed of, in the direction from a surface in contact with the substrate layer to a free surface, a polyethylene glycol succinimidyl ester layer and a polyethylene glycol amine layer that are laminated in this order;
preferably, the adhesive coating is composed of, in the direction from a surface in contact with the substrate layer to a free surface, a polyethylene glycol amine layer and a polyethylene glycol succinimidyl ester layer that are laminated in this order.

4. The PEG bicomponent self-adhesive absorbable biological patch according to any one of claims 1 to 3, wherein the substrate layer is the biological material substantially containing a collagen protein; preferably is submucosa, dermis, pericardium, collagen, or gelatin; more preferably is decellularized small intestinal submucosa, decellularized dermis or decellularized pericardium;
preferably, the substrate layer has a thickness of 0.01 mm to 1 mm, more preferably 0.05 mm to 0.5 mm;
preferably, the substrate layer is a homogeneous layer;
preferably, the substrate layer is in the form of a laminate formed by two or more separated layers; and/or
preferably, the substrate layer is composed of 2 to 9 layers of the decellularized small intestinal submucosa.

5. A combination product of a PEG bicomponent self-adhesive absorbable biological patch, comprising a substrate layer according to claim 1 or 4 and an adhesive coating according to any one of claims 1 to 3, which are packaged respectively.

6. A kit comprising an individually packaged substrate layer, and an individually packaged precursor solution composition for forming an adhesive coating;
wherein the substrate layer is as set forth in claim 1 or 4;
the precursor solution composition comprises a polyethylene glycol succinimidyl ester solution and a polyethylene glycol amine solution that are present independently, the polyethylene glycol succinimidyl ester solution has a concentration of 5.0 to 30.0 wt.%, preferably has a concentration of 10.0 to 20.0 wt.%, the polyethylene glycol amine solution has a concentration of 0.2 to 20.0 wt.%, preferably has a concentration of 1.0 to 15.0 wt.%, and solvents of both solutions are an aqueous solution;
preferably, the precursor solution composition further comprises an additive solution or a solid additive that is present independently.

7. A method of preparing a PEG bicomponent self-adhesive absorbable biological patch according to any one of claims 1 to 4, wherein a polyethylene glycol succinimidyl ester solution and a polyethylene glycol amine solution are coated on a substrate layer; the polyethylene glycol succinimidyl ester solution has a concentration of 5.0 to 30.0 wt.%, preferably has a concentration of 10.0 to 20.0 wt.%, the polyethylene glycol amine solution has a concentration of 0.2 to 20.0 wt.%, preferably has a concentration of 1.0 to 15.0 wt.%, and solvents of both solutions are an aqueous solution;
preferably, the polyethylene glycol succinimidyl ester solution and the polyethylene glycol amine solution are alternately coated in turn;
preferably, after a first polyethylene glycol solution is coated, the coated first polyethylene glycol solution is subjected to dehydration treatment before coating a second polyethylene glycol solution; and
preferably, after completion of all coating, a resulting self-adhesive absorbable biological patch is subjected to dehydration treatment.

8. A therapeutic agent delivery device for sealing a tissue surface and releasing one or more therapeutic agents to a target site, the device comprising: (i) a PEG bicomponent self-adhesive absorbable biological patch according to any one of claims 1 to 4, and (ii) a plaster loaded with one or more therapeutic agents, wherein the area of the plaster is smaller than the area of the PEG bicomponent self-adhesive absorbable biological patch, and the plaster is located on a free surface of an adhesive coating of the PEG bicomponent self-adhesive absorbable biological patch.

9. Use of a PEG bicomponent self-adhesive absorbable biological patch according to any one of claims 1 to 4 in the preparation of a tissue sealing material.
